# EUROPEAN PATENT APPLICATION

(11) **EP 3 184 650 A1**
(43) Date of publication of application: **28.06.2017**
(21) Application number: 16205850.7
(22) Date of filing: 21.12.2016
(51) Int. Cl.: C12Q 1/68

(54) **PERSONALISED COSMETIC METHOD FOR THE PREVENTION AND THE TREATMENT OF SKIN CHRONO- AND PHOTO-AGING**

(30) Priority: 23.12.2015 IT UB20159293
(71) Applicant: MDM Industrial S.r.l., 40010 Sala Bolognese (BO) (IT)
(72) Inventor: Montanari, Federico, 40010 Sala Bolognese BO (IT)
(74) Representative: Asensio, Raffaella Consuelo

(57) **Abstract**

The present patent application describes a customized cosmetic method for the prevention and treatment of time- and light-induced skin ageing (chronological and photo-ageing), wherein the selection of the formulation to be used is made based on the correlation between a genetic risk score and the distinctive parameters of skin ageing.

## Description

### FIELD OF THE INVENTION

The present patent application relates to a cosmetic method for the prevention and treatment of time- and light-induced skin ageing, customized based on an individual's genetic profile.

### BACKGROUND ART

Skin ageing is a process of multifactorial etiology which involves intrinsic factors, of a genetic and hormonal origin, and extrinsic factors, environmental and nutritional in nature.

The signs of ageing, which are visible at the skin level, are the local manifestation of the global senescence process, which is characterized by an alteration of the biochemical properties of the skin and is in turn generated by the combination of intrinsic and extrinsic factors, which are responsible for starting the degenerative processes of chronological and photo-ageing.

Also known as biological ageing, chronological ageing determines cell hypoactivity, i.e. a continuous and progressive slowing down of cell repair and renewal processes, resulting in a decreased effectiveness thereof.

Photo-ageing is characterized by an activation of oxidative stress phenomena, and thus by cell hyperactivity, whose main outcome is damage to nucleic acids, proteins and lipids.

Chronological ageing and photo-ageing act synergistically in generating the typical manifestations of skin ageing.

The structural alterations responsible for the visible signs of skin ageing mainly affect the surface layers of the skin: the increase in keratinocyte terminal differentiation determines a progressive thickening of the stratum corneum due to an accumulation of dead cells at the surface level, thus forming a compact matrix which alters skin hydration functions and gives the skin a dry and wrinkled appearance (Velarde et al. Aging (2012) Jan; 4(1): 3-12); on the other hand, a reduced collagen and elastin production causes dermal thinning, the degeneration of which results in a reduction in skin elasticity and turgor (Naylor et al. Maturitas (2011) Jul; 69(3): 249-56)

Sunlight exposure is one of the most important extrinsic factors in the photo-ageing process, i.e. an acceleration of the senescence process; light-induced genetic damage is in fact responsible for the increased expression of inflammatory cytokines, which are involved in oxidative stress phenomena and in the generation of phenotypes of accelerated skin ageing and cell senescence (Velarde et al. Aging (2012) Jan; 4(1): 3-12).

Due to involvement of genetic and environmental factors, skin ageing may be regarded as a chronic degenerative disease, wherein the combination of intrinsic and extrinsic factors plays an important role in altered regenerative, structural and defensive ability of the skin.

The importance of genetic variability for the development of complex diseases is well known in the art, which in recent years has highly focused on the role of genes and variants thereof in the onset of specific diseases.

Modifications affecting a coding gene may involve the production of proteins with different functionality, characterized by primary and tertiary structures different from the expected ones and potentially responsible for an individual's predisposition to certain diseases.

Among these modifications, single nucleotide polymorphisms (SNPs) are the most common type of genetic modification in the population; in fact, these are genetic material alterations affecting a single nucleotide, such that the polymorphic allele is present with a frequency greater than 1% in total in the reference population (http://ghr.nlm.nih.gov/handbook/genomicresearch/snp).

With reference to chronological ageing, the modifications affecting genes that encode for type 1 collagen (COL1A1) and elastin (ELN) are among the most studied factors of individual variability.

Type 1 collagen is the main structural component of the extracellular matrix of the dermis and its decrease in quality and quantity is directly involved in tissue relaxation phenomena which are typical of senescence.

Several studies have shown that common polymorphisms affecting the COL1A1 gene may change the expression of the above-mentioned protein, consequently altering the production and turnover thereof (Mann et al. J Clin Invest. (2001) Apr; 107(7): 899-907).

Elastin is a structural protein of connective tissue and is the main component of the elastic fibres making up the dermis.

There are polymorphisms associated to the ELN gene that code for proteins having altered mechanical properties, therefore responsible for an increased risk of impairment of skin elasticity (Hanon et al. Hypertension (2001) 38(5): 1185-9).

With reference to photo-ageing, there are various factors of genetic variability that can take part in degenerative metabolic processes.

Metalloprotease 3 (also referred to as MMP3) is a protease involved in the degradation of components making up the extracellular matrix of the dermis and in the tissue remodelling process, commonly activated during inflammatory phenomena.

Under conditions of sunlight exposure, skin inflammation or oxidative stress, the above-mentioned metalloprotease MMP3 activates, taking part in the degradation process of collagen fibres and elastic fibres forming the dermis; MMP3 enzyme activity may be modulated by genetic polymorphisms as shown in the literature (Vierkötter et al. J Invest Dermatol. (2015) May; 135(5): 1268-74).

Free radicals (ROS = Reactive Oxygen Species) are highly reactive substances derived from molecular oxygen that can damage DNA and cell structures, altering metabolic processes.

Poor diet, stress, exposure to cigarette smoke and pollutants are just some of the factors involved in the increase in cell production of reactive oxygen species; our bodies are physiologically equipped with protection systems against ROS, wherein the enzymes Superoxide Dismutase 2 (SOD2), Glutathione Peroxidase 1 (GPX1) and Catalase (CAT) play a key role in the transformation of radical species into inert species, which can be easily removed.

It is known that the variability caused by the presence of polymorphisms of genes coding for these proteins modulates enzyme activity resulting in a different individual susceptibility to oxidative stress (Bastaki et al. Pharmacogenet Genomics (2006); 16(4): 279-86).

Similarly, allelic variants of genes coding for cytokines interleukin-1β (IL1B) and tumor necrosis factor-α (TNFA) may determine a different susceptibility to inflammatory stimuli (Buchs et al. Genes Immun. 2001 Jun; 2(4): 222-8; Wilson et al. Immunol 94 (1997), pp. 3195-3199; Braun Neurosci Lett. (1996) Sep 6; 215(2): 75-8).

### SUMMARY OF THE INVENTION

The Applicant has now surprisingly found that it is possible to create a customized cosmetic method for the prevention and treatment of chronological and photo-ageing, wherein the selection of the formulation to be used is made based on the correlation between a genetic risk score and the distinctive parameters of skin ageing.

Said genetic risk score is built taking into account the contribution of each of the single nucleotide polymorphisms involved in the metabolic processes of the skin, by scoring it based on the impact that said polymorphism has on the processes of chronological and photo-ageing.

### DESCRIPTION OF THE FIGURES

Figure 1: shows the correlation between genetic risk score GRS and skin elasticity.
Figure 2: shows the correlation between genetic risk score GRS and thickness of the stratum corneum.
Figure 3: shows the percentage variations obtained with the customized cosmetic anti-ageing method, compared to treatment with radiofrequency. The left column relates to percentage variations in thickness of the stratum corneum; the right column relates to percentage variations in skin elasticity.

### DETAILED DESCRIPTION OF THE INVENTION

For the purposes of the present invention, cosmetic method means a method of care and maintenance of a person's appearance, wherein the individual does not receive any surgical treatment, whereas they are treated by applying a topical formulation.

Customized cosmetic method means the above-described method, wherein the formulation being used has specific components which take into account the individual features of the subject to be treated and in particular the genetic predisposition the individual has towards the processes causing skin ageing.

The Applicant has now surprisingly found that said individual genetic predisposition may be determined using the correlation between a personal genetic risk score and the distinctive parameters of chronological and photo-ageing.

Within the scope of the present invention, genetic risk score or GRS means an objective numerical quantification of the patient's genotype which gives information about the subject's sensitivity towards the processes of chronological and photo-ageing.

Said GRS is built based on the patient's DNA sampling and genotyping, i.e. characterization of an individual's genetic constitution by identifying specific polymorphisms affecting the genes COL1A1, ELN involved in the chronological ageing process, and specific polymorphisms affecting the genes CAT, GPX, MnSOD2, IL-1B, TNF-A, MMP3 involved in the photo-ageing process.

More specifically, the polymorphisms included in the assessment of an individual's sensitivity to chronological and photo-ageing are preferably as follows: COL1A1 rs1800012 is involved in the turnover process of type I collagen (Mann et al. 2001, J. Clin. Invest. 107: 899-907), the main component of the skin; ELN rs2071307 affects the mechanical properties of the protein elastin, responsible for elasticity of the dermis (He et al. PLoS One. 2012; 7(9): e46130); MMP3 rs3025058 is involved in membrane matrix remodelling, by modulating protease activity (Souslova et al. PLoS One. 2010; 5(3): e9902); CAT rs1001179, GPX rs1050450 and MnSOD2 rs1799725 modulate antioxidant capability (Bastaki et al. Pharmacogenetics and Genomics 2006, 16:279-286); IL-1B rs1143634 and TNF-A rs1800629 determine a different susceptibility to inflammatory stimulus (Buchs et al. Genes and Immunity (2001) 2, 222-228; Menges et al. Crit Care Med. 2008 May; 36(5): 1456-62).

Within the scope of the present invention, distinctive parameters of chronological and photo-ageing mean skin properties, experimentally measurable, which are subject to significant modification over time and whose variation can give an indication about the progression of the senescence process.

Time- and light-induced skin ageing is measurable, at a skin level, by said distinctive parameters of chronological and photo-ageing which, in the cosmetic method of the present invention, are preferably skin elasticity and thickness of the stratum corneum, measured on the patient's cheek by impedance analysis.

Impedance analysis means a non-invasive method to analyze body composition thanks to the detection of impedance, i.e. the body's resistance to the passage of a low-frequency alternating electric current. The present technique is based on the physical principle that biological tissues act as conductors (intra- and extracellular fluids), semiconductors or insulators (bone and fat).

Starting from the body impedance value, i.e. from the measurement of the resistance body tissues provide against current, it is possible to determine the patient's contents of body water, lean mass, fat mass, and the basal metabolic rate.

The rationale of the present invention lies in that there exists significant correlation between genetic risk score and progression of the specific skin parameters, providing the basis for assessing an individual's genetic predisposition to skin ageing.

Advantageously, there is a correlation between time- and light-induced skin ageing, measured at the skin level by the specific parameters of skin elasticity and thickness of the stratum corneum, and said genetic risk score GRS.

The analysis of correlation between genetic variability and skin scoring is better described in the experimental section of the present patent application, where evidence is given about dependence between the two variables.

Advantageously, said genetic risk score GRS is inversely related to skin elasticity and directly related to thickness of the stratum corneum.

Each polymorphism analyzed is assigned an arbitrary numerical value which quantifies the impact of said genetic variation on individual susceptibility to ageing, based on the information available in the above-cited literature.

Said arbitrary numerical value equals 1 if the detected genotype contains two alleles which are considered unfavourable, and thus associated with increased susceptibility to skin ageing; equals 0 if a single unfavourable allele is detected; equals -1 if no unfavourable allele is detected.

The correlation between the assigned numeric value and genotype is exemplified in Table 1.

**Table 1**

| **Gene** | **Ageing process** | **SNP** | **1 Value** | **0 Value** | **-1 Value** |
|---|---|---|---|---|---|
| COL1A1 | Chronological | rs1800012 | TT | GT | GG |
| ELN | Chronological | rs2071307 | AA | AG | GG |
| CAT | Photo-ageing | rs1001179 | GG | AG | AA |
| GPX | Photo-ageing | rs1050450 | TT | TC | CC |
| MnSOD2 | Photo-ageing | rs1799725 | CC | TC | TT |
| IL-1B | Photo-ageing | rs1143634 | TT | TC | CC |
| TNF-A | Photo-ageing | rs1800629 | GG | AG | AA |
| MMP3 | Photo-ageing | rs3025058 | 00 | T0 | TT |

For each patient, the GRS is calculated by means of an addition model, by summing up the scores obtained for each of the single nucleotide polymorphisms identifiable in the patient's genome and forming part of Table 1, according to the "single SNP based test" model described in the work of Ballard et al. (Genet Epidemiol. 2010 April; 34(3): 201-212).

The genetic risk score described in the present invention can ideally take any value from -8 to 8, wherein GRS = -8 indicates the lowest genetic predisposition to skin ageing, whereas GRS = 8 indicates the highest susceptibility.

Once the subject's GRS is calculated, individual sensitivity to chronological and photo-ageing is determined by comparison between the GRS of each patient and Table 2, below.

**Table 2**

| **AREA** | **SENSITIVITY** | **GRS VALUES** | **FREQUENCY** |
|---|---|---|---|
| Chronological ageing | LOW | 0 ≤ GRS ≤ -2 | 36.72% |
| | INTERMEDIATE | = 1 | 40.86% |
| | HIGH | = 2 | 22.42% |
| Photo-ageing | LOW | -1< GRS < -6 | 41.7% |
| | INTERMEDIATE | = 0 | 26.1% |
| | HIGH | 1 ≤ GRS ≤ 6 | 32.2% |

The present Table 2 was built combining the GRS calculation with the frequencies of polymorphisms under examination in the population of European origin, as can be derived from data published in the literature and collected in the Hapmap database (www.hapmap.org) and in databases that can be browsed through on the National Centre for Biotechnology Information website (www.ncbi.nlm.nih. gov/snp)

The GRS distribution, based on the genotype frequencies of the analyzed polymorphisms, allows grouping subjects' genotypes in 3 arbitrary categories of sensitivity to chronological and photo-ageing.

For each GRS, therefore, there exists a combination of two cosmetic compositions, respectively suitable for chronological and photo-ageing, standardizing product selection based on the categories of low, intermediate and high sensitivity.

For the purposes of the present invention, the cosmetic method uses compositions prepared in the form of inert conductive gels, enriched with specific active ingredients and applied to the patient through the use of a radiofrequency device, which facilitates deep absorption of the active ingredients.

The cosmetic compositions are divided into six formulations: three of which were studied for the customized prevention and treatment of the effects related to chronological ageing; and three of which contain specific active ingredients for treating the effects of photo-ageing.

Before applying the cosmetic composition, the cosmetic method of the present invention involves the application of a preparatory gel, specially studied to stimulate in advance the tissue to be treated and promote transdermal penetration of the active ingredients.

The preparatory gel used in the present cosmetic method comprises Aqua [Water], Propylene Glycol, Sodium gluconate, Ammonium acryloyldimethyltaurate / VP copolymer, Benzyl alcohol, Mentha piperita extract [Mentha piperita (Peppermint) extract], Coceth-7, PPG-1-PEG -9 lauryl glycol ether, Dehydroacetic acid, Parfum [Fragrance], PEG-40 hydrogenated castor oil.

The cosmetic method for the prevention and treatment of chronological ageing uses cosmetic compositions which vary in type and concentration of the active ingredients and which have been formulated based on the genetic predisposition to sensitivity to chronological ageing, as reported in Table 3.

For skin that does not show an impairment in terms of collagen and elastin production, the cosmetic composition will be aimed at increasing skin hydration and nourishment in order to allow for good cell function.

On the contrary, if genetic testing detects a potential impairment of expression of proteins responsible for maintaining dermal tone, the composition of the gel used for the prevention and treatment of chronological ageing is aimed at stimulating the metabolism of fibroblasts, which are cells responsible for synthesizing collagen and elastin.

**Table 3**

| **SENSITIVITY** | **COMPOSITION** |
|---|---|
| LOW | Aqua [Water], Propylene glycol, Saccharide isomerate, Ammonium acryloyldimethyltaurate/VP copolymer, Sodium gluconate, Benzyl alcohol, Coceth-7, PPG-1-PEG-9 lauryl glycol ether, Dehydroacetic acid, Parfum Fragrance], Hydrolyzed soy protein, PEG-40 hydrogenated castor oil. |
| INTERMEDIATE | Aqua [Water], Propylene glycol, Glycerin, Ammonium acryloyldimethyltaurate/VP copolymer, Saccharide isomerate, Sodium gluconate, Benzyl alcohol, Palmitoyl tripeptide-5, Coceth-7, PPG-1-PEG-9 lauryl glycol ether, Dehydroacetic acid, Parfum [Fragrance], PEG-40 hydrogenated castor oil. |
| HIGH | Aqua [Water], Propylene glycol, Glycerin, Ammonium acryloyldimethyltaurate/VP copolymer, Saccharide isomerate, Sodium gluconate, Benzyl alcohol, Fagus sylvatica bud extract, Palmitoyl tripeptide-5, Coceth-7, PPG-1-PEG-9 lauryl glycol ether, Dehydroacetic acid, Parfum [Fragrance], PEG-40 hydrogenated castor oil, Lecithin, Tocopherol, Ascorbyl palmitate, Citric acid. |

The active ingredients used for the formulation of these cosmetic compositions have been widely studied for their anti-ageing features.

The *Fagus sylvatica* (Beech tree bud) extract contains a high quantity of phytostimulines, which are molecules known for their important effect of metabolism activation and which have shown to significantly stimulate protein synthesis of keratinocyte cultures in vitro.

In vivo studies have shown that the Fagus sylvatica extract increases skin smoothness, reducing the depth of wrinkles and improving skin hydration (Benhaïm et al. Söfw. seifen, ole, fette, wachse issn 0173-5500, 1995, vol. 121, no1, pp. 3-7).

The sugar moisturizing factor is a carbohydrate complex similar to that contained in the skin, which acts by binding to amino acid lysine residues exposed by keratins, attracting water (Padberg et al. J. Soc. Cosmetics Chemists (1972), 23, 271-279) and providing deep and long-lasting hydration, contributing to maintaining the skin barrier function.

Palmitoyl Tripeptide-3 is a synthetic peptide, capable of penetrating the skin and increasing collagen production by fibroblasts (Lupo & Cole. Dermatologic Therapy, (2007) Vol. 20, 343-349), mimicking the action of thrombospondin-1, a multifunctional protein that activates the transforming growth factor beta (TGF-β) (Frazier. Curr. Opin. Cell Biol. (1991) 3,792-799).

It has been shown that peptides or proteins naturally extracted from soy beans may inhibit the action of extracellular matrix proteinases, helping to maintain the integrity of skin structure (Centerchem, Inc., Norwalk, CT, USA).

The use of proteins hydrolyzed from soy beans increases fibroblast trophism (Sudel et al. Photochemistry and Photobiology, (2005) Vol. 81, No. 3, 581-587) promoting collagen and glycosaminoglycan synthesis (Andre-Frei et al. International Journal of Cosmetic Science, (1999) Vol. 21, No. 5, 299-311).

Moreover, the extract of soy bean hydrolyzed proteins contains antioxidant peptides (Chen et al. Journal of Agricultural and Food Chemistry, (1998) Vol. 46, No. 1, 49-53) with a protective action toward lieleic acid peroxidation, neutralizing the effects of peroxynitrite and oxygen free radicals (Takenaka et al. Bioscience, Biotechnology, and Biochemistry. (2003) Vol, 67. No. 2, 278-283).

The cosmetic method for the prevention and treatment of photo-ageing uses cosmetic compositions containing active ingredients capable of fighting and preventing the signs of photo-induced skin ageing, formulated based on the genetic predisposition to sensitivity to dermatoheliosis (respectively: low, intermediate, high).

Such compositions, shown in Table 4, act preventively protecting the skin from photo-induced damage, while maintaining an effective moisturizing action over time, preventing damage from free radicals on cell membranes and DNA, reducing damage from solar radiation and improving the sensation of healthy skin, neutralizing the sensory manifestations of inflammation.

**Table 4**

| **SENSITIVITY** | **COMPOSITION** |
|---|---|
| LOW | Aqua [Water], Propylene glycol, Mentha piperita extract [Mentha piperita (Peppermint) extract], Ammonium acryloyldimethyltaurate/VP copolymer, Saccharide isomerate, Sodium gluconate, Benzyl alcohol, Pyrus malus extract [Pyrus malus (Apple) fruit extract], Coceth-7, PPG-1-PEG-9 lauryl glycol ether, Dehydroacetic acid, Parfum [Fragrance], PEG-40 hydrogenated castor oil, Lecithin, Tocopherol, Ascorbyl palmitate, Citric acid. |
| INTERMEDIATE | Aqua [Water], Propylene glycol, Mentha piperita extract [Mentha piperita (Peppermint) extract], Hydrolyzed grape fruit, Ammonium cryloyldimethyltaurate/VP copolymer, Sodium gluconate, Benzyl alcohol, Coceth-7, PPG-1-PEG-9 lauryl glycol ether, Dehydroacetic acid, Parfum [Fragrance], PEG-40 hydrogenated castor oil, Lecithin, Tocopherol, Ascorbyl palmitate, Citric acid. |
| HIGH | Aqua [Water], Propylene glycol, Mentha piperita extract [Mentha piperita (Peppermint) extract], Hydrolyzed grape fruit, Ammonium acryloyldimethyltaurate/VP copolymer, Sodium gluconate, Benzyl alcohol, Coceth-7, PPG-1-PEG-9 lauryl glycol ether, PEG-40 hydrogenated castor oil, Dehydroacetic acid, Oleyl alcohol, Parfum [Fragrance], Zanthoxylum bungeanum fruit extract, Lecithin, Tocopherol, Ascorbyl palmitate, Citric acid. |

Peppermint is a perennial herbaceous, highly aromatic plant, with stolons, belonging to the Labiatae family (Lamiaceae) and to the genus Mentha. In vitro studies have shown that peppermint possesses significant antimicrobial, antiviral, antioxidant (particularly due to eriocitrin) and antiallergic activity, and that some of the flavonoid glycosides contained therein, such as luteolin-7-O-rutinoside, have a powerful effect on histamine release triggered by reactions of the antigen/antibody kind.

Furthermore, menthol may significantly suppress the production of inflammatory mediators such as leukotrienes (LT) B4, prostaglandin (PG) E2, and interleukin (IL)-β2.

The *Pyrus malus* extract is a natural antioxidant, rich in flavonoids and chalcones, which preserves skin healthiness and vitality, thereby limiting the mechanisms of cell protein and enzyme oxidation, thus reducing the risk of DNA degradation in vitro.

Zanthalene, extracted from *Zanthoxylum bungeanum,* is an active ingredient capable of ameliorating wrinkles. The lipophilic hydroxyalkamides provided in the Zanthalene extract act transiently and reversibly on synaptic neurotransmission of Na +-dependent channels, affecting heat and touch sensitivity, reducing skin discomfort such as itching.

The *Vitis Vinifera* extract protects the skin against UV-light over-exposure, environmental pollutants and adverse weather conditions. Its antioxidant effectiveness is related to the abundance of phenols, anthocyanosides and catechins present in the skin of red grapes, showing an antimutagenic, antioxidant, antiinflammatory and free radical-neutralizing activity. The Vitis Vinifera extract shows inhibitory activity against metalloproteinases causing dermal degeneration.

### ANALYSIS OF THE CORRELATION BETWEEN GENETIC RISK SCORE AND SKIN PARAMETERS

The analysis of the correlation between genetic risk score and objective skin parameters was obtained by a study involving 100 Italian volunteers, between 21 and 66 years of age (23 males and 77 females) belonging to phototype 2 and 3 (Fitzpatrick).

DNA sampling was conducted using a buccal swab on oral mucosal shedding cells and analyzed by Real-Time PCR, using standard methods.

The patient's GRS was calculated according to the methods described in the present patent application.

In addition to the genetic component, components including age, light exposure, smoke and hydration were assessed in the reference sample as extrinsic factors affecting the ageing process; more precisely, light exposure was determined by the use of the SEBI questionnaire (Jennings et al. JEADV 2013, 27, 706-715)*,* whereas the parameters established by Singh et al. were used for assessing smoking habits (Singh et al. Skin Res Technol. 2013 Aug;19(3):207-12. doi: 10.1111/srt.12043).

The experimental determination of skin degeneration was carried out by measuring skin elasticity and thickness of the stratum corneum, taken as objective parameters of the senescence process; each volunteer was subjected to skin testing using the Skin Tester device (Selenia, Italy), which is based on the principle of impedance analysis.

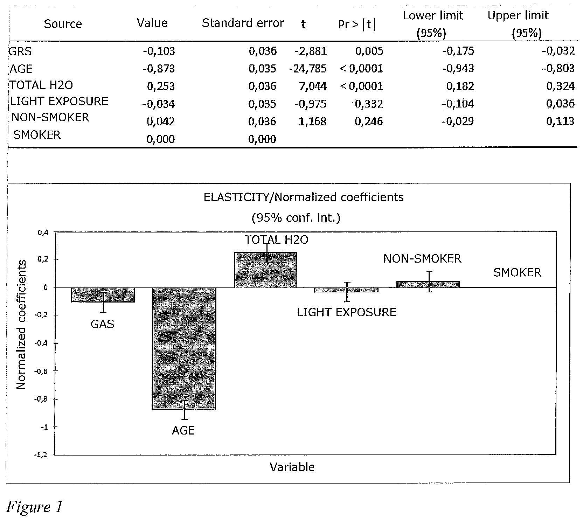

Results were expressed as mean values of two measurements, conducted on each of the volunteer's cheeks and are shown in the graph of Figure 1.

The correlation between the genetic component measured with GRS and skin parameters was evaluated by analysis of covariance (ANCOVA), to assess the impact of different skin ageing factors on elasticity and stratum corneum.

Figure 1 shows that there is significant inverse correlation between GRS and elasticity, indicating that increasing the number of variables related to ageing decreases elasticity.

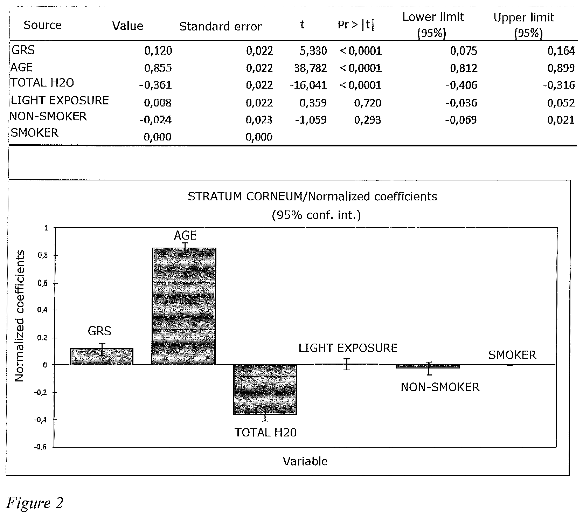

Figure 2 shows that there is significant correlation between GRS and thickness of the stratum corneum, indicating that increasing the number of variables related to ageing increases the thickness of said layer.

### EXAMPLE

### VALIDATION OF THE CUSTOMIZED COSMETIC METHOD

The study included 9 subjects aged between 26 and 49 years, who were subjected to the cosmetic method described in the present invention.

The cosmetic compositions, selected based on the degree of personal susceptibility to ageing, were applied to one half of the patient's face by a radiofrequency device, commercially known by the name of Genotechnology-1®.

The Genotechnology-1® device is equipped with a specific handpiece capable of delivering exogenous heat which, when associated with endogenous heat generated from the passage of the radiowave into the dermis (the principle of radiofrequency), increases penetration of the active ingredients through the skin barrier (Clarys et al.(1998) Eur. J Pharm. Biopharm. 46, 279-283; Akomeah et al.(2004) Eur. J. Pharm Sci. 21, 337-345)

The protocol based on half-face treatment was selected in order to eliminate individual variability, caused by exposure to different environmental pressures.

The remaining half face was regarded as the control and treated by radiofrequency using the same device (which allows for the two treatment modes), applying a standard ultrasound conductive gel (placebo) and using the same delivery specifications as the treated part.

Treatment was carried out in 10 sessions twice weekly; on one of the half faces the cosmetic compositions selected based on the patient's susceptibility were applied, according to an application procedure which implies, for each session, the following order of application: preparatory gel, chronological ageing gel, photo-ageing gel.

Skin elasticity and thickness of the stratum corneum were measured on the cheek of the subjects involved in the study, using a Skin Tester device (Selenia, Italy), which uses the principle of impedance analysis for assessing the above-cited parameters.

Each subject was subjected to 2 measurements, for both the right and the left cheek, and the mean value of the measurements was calculated. All statistical analyses were performed using the XLSTAT® (Addinsoft) software.

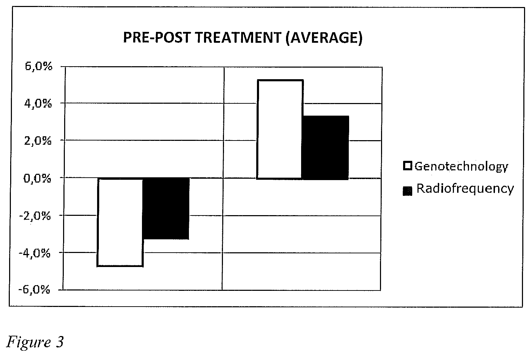

Results show that there is statistically significant difference in the results obtained after treatment, with greater efficiency of the anti-ageing treatment with Genotechnology-1 as compared to the radiofrequency treatment, for all the parameters under examination (Figure 3).

The average (percentage) increases obtained with the anti-ageing protocol are summarized in Table 5.

**Table 5**

| **Anti-ageing** | | |
|---|---|---|
| | STRATUM CORNEUM | ELASTICITY |
| AVERAGE | -4.7% | 5.3% |
| SD | 2.9% | 2.1% |

| **Radiofrequency** | | |
|---|---|---|
| | STRATUM CORNEUM | ELASTICITY |
| AVERAGE | -3.3% | 3.3% |
| SD | 2.4% | 2.1% |

The differences between groups were assessed using a Student's t-test and were all found to be highly significant (P <0.01%).

The genetic customization of the compositions used for the prevention and treatment of skin alterations induced by chronological and photo-ageing has made it possible to create a customized cosmetic method with significantly greater effectiveness than the generic treatment based solely on the principle of radiofrequency.

## Claims

1. Customized cosmetic method for use in the treatment and prevention of time- and light-induced skin ageing (chronological and photo-ageing), comprising the steps of:
a. A patient's DNA sampling and genotyping for detecting polymorphisms affecting the genes COL1A1, ELN involved in the chronological ageing process, and the polymorphisms of CAT, GPX, MnSOD2, IL-1B, TNF-A, MMP3 involved in the photo-ageing process.
b. Defining the patient's genetic risk score GRS, by scoring each of the genetic polymorphisms of the above item a., wherein said score equals 1 if the detected genotype contains two alleles regarded as unfavourable, therefore associated with greater susceptibility to skin ageing; 0 value if a single unfavourable allele is detected; -1 value if no unfavourable allele is detected.
c. Assigning a total score to the patient's genotype, obtained by summing up the scores assigned at b.
d. Identifying the patient's sensitivity to chronological and photo-ageing, by comparison between the GRS obtained at c. and the following table of genotype frequencies:
| AREA | SENSITIVITY | GRS VALUES | FREQUENCY |
|---|---|---|---|
| Chronological ageing | LOW | 0 ≤ GRS ≤ - 2 | 36.72% |
| | INTERMEDIATE | =1 | 40.86% |
| | HIGH | =2 | 22.42% |
| Photo-ageing | LOW | -1< GRS ≤ - 6 | 41.7% |
| | INTERMEDIATE | =0 | 26.1% |
| | HIGH | 1 ≤ GRS ≤ 6 | 32.2% |
e. Assigning each GRS the combination of two cosmetic compositions, suitable for the level of susceptibility to chronological and photo-ageing,
wherein
said time- and light-induced skin ageing, measured by parameters distinctive of time- and light-induced skin ageing, selected between skin elasticity and thickness of the stratum corneum, is related to said GRS.

2. Cosmetic method according to claim 1, wherein said genetic risk score GRS is inversely related to said skin elasticity and directly related to said thickness of the stratum corneum.

3. Cosmetic method according to claim 1 or 2 for the treatment of chronological ageing, wherein a cosmetic composition comprising Aqua, Propylene glycol, Saccharide isomerate, Ammonium acryloyldimethyltaurate/VP copolymer, Sodium gluconate, Benzyl alcohol, Coceth-7, PPG-1-PEG-9 lauryl glycol ether, Dehydroacetic acid, Parfum, Hydrolyzed soy protein, PEG-40 hydrogenated castor oil is applied to patients with a GRS between 0 and -2 inclusive.

4. Cosmetic method according to claim 1 or 2 for the treatment of chronological ageing, wherein a cosmetic composition comprising Aqua, Propylene glycol, Glycerin, Ammonium acryloyldimethyltaurate/VP copolymer, Saccharide isomerate, Sodium gluconate, Benzyl alcohol, Palmitoyl tripeptide-5, Coceth-7, PPG-1-PEG-9 lauryl glycol ether, Dehydroacetic acid, Parfum, PEG-40 hydrogenated castor oil is applied to patients with a GRS of 1.

5. Cosmetic method according to claim 1 or 2 for the treatment of chronological ageing, wherein a cosmetic composition comprising Aqua, Propylene glycol, Glycerin, Ammonium acryloyldimethyltaurate/VP copolymer, Saccharide isomerate, Sodium gluconate, Benzyl alcohol, Fagus sylvatica bud extract, Palmitoyl tripeptide-5, Coceth-7, PPG-1-PEG-9 lauryl glycol ether, Dehydroacetic acid, Parfum, PEG-40 hydrogenated castor oil, Lecithin, Tocopherol, Ascorbyl palmitate, Citric acid is applied to patients with a GRS of 2.

6. Cosmetic method according to claim 1 or 2 for the treatment of photo-ageing, wherein a cosmetic composition comprising Aqua, Propylene glycol, Mentha piperita extract [Mentha piperita (Peppermint) extract], Ammonium acryloyldimethyltaurate/VP copolymer, Saccharide isomerate, Sodium gluconate, Benzyl alcohol, Pyrus malus extract [Pyrus malus (Apple) fruit extract], Coceth-7, PPG-1-PEG-9 lauryl glycol ether, Dehydroacetic acid, Parfum, PEG-40 hydrogenated castor oil, Lecithin, Tocopherol, Ascorbyl palmitate, Citric acid is applied to patients with a GRS between -1 and -6 inclusive.

7. Cosmetic method according to claim 1 or 2 for the treatment of photo-ageing, wherein a cosmetic composition comprising Aqua, Propylene glycol, Mentha piperita extract [Mentha piperita (Peppermint) extract], Hydrolyzed grape fruit, Ammonium cryloyldimethyltaurate/VP copolymer, Sodium gluconate, Benzyl alcohol, Coceth-7, PPG-1-PEG-9 lauryl glycol ether, Dehydroacetic acid, Parfum, PEG-40 hydrogenated castor oil, Lecithin, Tocopherol, Ascorbyl palmitate, Citric acid is applied to patients with a GRS of 0.

8. Cosmetic method according to claim 1 or 2 for the treatment of photo-ageing, wherein a cosmetic composition comprising Aqua, Propylene glycol, Mentha piperita extract [Mentha piperita (Peppermint) extract], Hydrolyzed grape fruit, Ammonium acryloyldimethyltaurate/VP copolymer, Sodium gluconate, Benzyl alcohol, Coceth-7, PPG-1-PEG-9 lauryl glycol ether, PEG-40 hydrogenated castor oil, Dehydroacetic acid, Oleyl alcohol, Parfum, Zanthoxylum bungeanum fruit extract, Lecithin, Tocopherol, Ascorbyl palmitate, Citric acid is applied to patients with a GRS between 1 and 6 inclusive.

9. Cosmetic method according to any one of claims 1 to 8, wherein, prior to the application of the cosmetic compositions, the skin is stimulated in advance with a preparatory gel.

10. Cosmetic method according to claim 9, wherein the cosmetic compositions and preparatory gel are applied to the patient by means of a radiofrequency device.
